# EUROPÄISCHE PATENTANMELDUNG

(11) **EP 2 275 080 A1**
(43) Veröffentlichungstag der Anmeldung: **19.01.2011**
(21) Anmeldenummer: 10004511.1
(22) Anmeldetag: 29.10.2005
(51) Int. Cl.: A61K 8/60, A61Q 19/00, C07H 13/06

(54) **Verfahren zur Herstellung von Kohlenhydratpartialestern**

(30) Priorität: 10.11.2004 DE 102004054432
(62) Teilanmeldung aus: 05805675.5
(71) Anmelder: Cognis IP Management GmbH, 40589 Düsseldorf (DE)
(72) Erfinder: Le Hen Ferrenbach, Catherine, 77100 Meaux (FR); Beuché, Marc, 91430 Vauhallan (FR); Roussel, Myriam, 77540 Lumigny (FR)

(57) **Zusammenfassung**

Vorgeschlagen wird ein solvensfreies Verfahren zur Herstellung von Kohlenhydratpartialestern mit einem durchschnittlichen Veresterungsgrad im Bereich von 1 bis 4 durch alkalikatalysierte Umesterung in Gegenwart eines Katalysator-Gemisches aus Alkalimetallcarbonat und Alkalimetallhypophosphit. Gegenstand sind auch die Verwendung der so erhaltenen Kohlenhydratpartialester sowie spezielle Formkörper auf Basis dieser Ester.

## Beschreibung

### Gebiet der Erfindung

Die Erfindung betrifft ein verbessertes Verfahren zur Herstellung von Kohlenhydratpartialestern durch Umesterung von Glykosen mit Fettsäureestern in Gegenwart von Emulgatoren und einem speziellen Katalysator-Gemisch sowie die Verwendung der nach dem Verfahren erhältlichen Stoffe.

### Stand der Technik

Kohlenhydratester, die auch häufig vereinfachend als "Zuckerester" bezeichnet werden, stellen Ester von Mono- oder Oligosacchariden, im weiteren Sinne auch von Zuckeralkoholen, mit organischen oder anorganischen Säuren dar. Kohlenhydratester besitzen ausgeprägte grenzflächenaktive Eigenschaften, weshalb man sie heute als eigenständige Verbindungsklasse (sogenannte Zuckertenside) betrachtet. Wegen ihrer guten dermatologischen und toxikologischen Verträglichkeit dienen Kohlenhydratester überwiegend als Emulgatoren zur Herstellung von Lebensmitteln und Kosmetika. Saccharosepolyester mit 6 bis 8 Fettsäureresten werden als vom Organismus nicht verwertbares Fettsubstitut in der Ernährung übergewichtiger Personen eingesetzt u. sollen außerdem LDL-Cholesterin im Darm binden. Im Stand der Technik ist eine Reihe von Verfahren zur Herstellung von Zuckerestern bekannt. Üblicherweise werden Glykosen in Gegenwart von alkalischen Katalysatoren und gegebenenfalls Emulgatoren mit Fettsäuremethylestern einer Umesterung unterworfen, die sowohl unter Zusatz von Lösungsmitteln als auch lösungsmittelfrei durchgeführt werden kann. Die solvensfreie Variante wird insbesondere dann bevorzugt, wenn die Zuckerester in der Lebensmittelindustrie eingesetzt werden sollen. Beispielsweise wird in der DE 4131505 ein Verfahren zur Aufarbeitung von Saccharosefettsäureestern beschrieben, die lösungsmittelfrei durch Umesterung von Saccharose mit Fettsäurealkylester in Gegenwart eines basischen Umesterungskatalysators hergestellt werden, wobei die nicht umgesetzte Saccharose bei einer Temperatur zwischen den Schmelzpunkten der eingesetzten Saccharose und des hergestellten Saccharoseesters abfiltriert und danach nicht umgesetzter Fettsäurealkylester aus dem Reaktionsgemisch abdestilliert wird. Gegenstand der EP 254376 ist ein solvensfreies Gesamtverfahren zur Herstellung von Polyolfettsäureestern, in denen weniger als die Hälfte der Hydroxylgruppen verestert ist, wobei intermediär ggf. ein Lösungsmittel zugegeben werden muss, um katalytisch aktive Polyol-Anionen zu bilden. Solvensfreie Prozesse zur Herstellung hoch veresterter Polyolfettsäureester sind auch Gegenstand der WO 99/38875**,** der EP 548272**,** der EP 550526**,** der EP 322971 und der EP 349059**.** Ein solvensfreier Prozess zur Herstellung Sucrosefettsäureester in Gegenwart eines basischen Katalysators ist Gegenstand der EP 885 898**:** Sucrose wird hier mit Fettsäurealkylestern bei 120 - 160 °C unter Druckbedingungen von 25 bis 100 mbar umgesetzt, die Temperatur dann im weiteren Reaktionsverlauf auf 90 - 130 °C abgesenkt und das resultierende Reaktionsprodukt ohne Zusatz eines Solvens filtriert. Dieses Verfahren liefert dunkelbraune viskose Rohprodukte, die in einem Folgeschritt gebleicht werden. Ein Verfahren zur Herstellung von Polyolpolyestem mit verbesserter Farbqualität ist Gegenstand der EP 349 221**:** hier werden C1-C3-Alkylfettsäureester mit einem Carbonylgehalt von weniger als 200 ppm mit einem Polyol in Gegenwart eines Katalysators umgesetzt und gleichzeitig der gebildete Alkohol dem Gleichgewicht entzogen. Aus der DE 19717968 ist ein Verfahren zur Herstellung von Kohlenhydratpartialestern bekannt, in dem man die alkalikatalysierte Umesterung in Gegenwart von Emulgatoren durchführt und zuerst ein katalytisch aktives System aus Alkalicarbonaten mit Fettsäureniedrigalkylestern bildet und dieses dann mit Glykosen und Kohlenhydratpartialestern als Emulgatoren umsetzt. Dieses Verfahren liefert ebenfalls noch relativ stark gefärbte Rohprodukte, die auch einen erhöhten Aschegehalt (DGF Methoden "Asche C III 1097) aufweisen. FR 9916213 beschreibt Gemische auf Basis von 10 bis 90 Gew.-% Zuckerestem und 90 bis 10 Gew.-% Kohlenwasserstoffen, die in kosmetischen Formulierungen eingesetzt werden. Pelletierte Zusammensetzungen dieser Gemische scheiden jedoch nach einiger Zeit Lagerung Öl aus.

Üblicherweise liefern die lösungsmittelfreien Verfahren ein komplexes Reaktionsgemisch, welches in erhöhtem Maße nicht abreagierte Ausgangsprodukte enthält, deren Abtrennung aufwendig ist. Diese Produkte sind oft sehr dunkel gefärbt. Farblich hellere Produkte erhält man gemäß einem Verfahren wie es in der EP 349221 beschrieben ist. Dieses setzt jedoch voraus, dass man vorgereinigte Fettsäurealkylester einsetzt, so dass ein zusätzlicher Reaktionsschritt notwendig ist.

Ziel der vorliegenden Erfindung war es, ein alternatives solvensfreies, wirtschaftliches Verfahren mit guten Ausbeuten und möglichst kurzen Reaktionszeiten zur Verfügung zu stellen, welches farblich verbesserte Rohprodukte mit verringertem Aschegehalt liefert und nicht die Vorreinigung der Ausgangsstoffe voraussetzt.

Überraschenderweise wurde festgestellt, dass eine signifikante Verfahrensverbesserung erreicht werden kann, wenn man als Katalysatorgemisch eine Mischung aus Alkalimetallcarbonat und Alkalimetallhypophosphit einsetzt.

### Beschreibung der Erfindung

Gegenstand der Erfindung ist ein solvensfreies Verfahren zur Herstellung von Kohlenhydratpartialestern mit einem durchschnittlichen Veresterungsgrad im Bereich von 1 bis 4 durch alkalikatalysierte Veresterung in Gegenwart eines Katalysator-Gemisches aus Alkalimetallcarbonat und Alkalimetallhypophosphit, wobei man
(a) zur Bildung eines katalytisch aktiven Systems wenigstens ein Alkalimetallcarbonat (a1) und wenigstens ein Fettsäurealkylester (a2) der Formel

   R¹CO-OR² (I)

   mischt, in der R¹CO für einen linearen oder verzweigten, gesättigten oder ungesättigten Acylrest mit 6 bis 30 Kohlenstoffatomen und R² für einen linearen oder verzweigten Alkylrest mit 1 bis 5 Kohlenstoffatomen steht, und
(b) zu der aus (a) resultierenden Mischung unter stetigem Rühren (b1) Glykosen mit 5 bis 12 Kohlenstoffatomen, (b2) Kohlenhydratpartialestern als Emulgatoren und (b3) Alkalimetallhypophosphit zugibt, so dass eine Dispersion resultiert, und dem resultierenden Gemisch unter stetigem Rühren bei Temperaturen bis zu 100° C bei einem Druck von bis zu 50 mbar Wasser entzieht und
(c) die Veresterungsreaktion bei einem Druck von bis zu 50 mbar und Temperaturen bis zu 125°C unter stetigem Rühren solange fortsetzt, bis der Gehalt an Fettsäurealkylestern der Formel (I) auf mindestens 8 Gew-% bezogen auf die Gesamtzusammensetzung abgefallen ist,
wobei die Schritte (a) bis (c) optional unter einer Schutzgasatmosphäre durchgeführt werden.

Die Katalysator-Kombination aus Alkalimetallcarbonat und Alkalimetallhypophosphit liefert im erfindungsgemäßen Verfahren farblich verbesserte Produkte in guten Ausbeuten und vergleichsweise kürzeren Reaktionszeiten, die einen geringeren Aschegehalt aufweisen. Zusätzlich wird die Bildung von Nebenprodukten, wie Seife reduziert. Als Schutzgas wird vorzugsweise Stickstoff eingesetzt. Der Einsatz von Schutzgasen liefert farblich noch hellere Produkte.

Im ersten Schritt (a) des Verfahrens entsteht ein Überzug von Fettsäureniedrigalkylester auf dem Alkalicarbonat, wodurch die Acylgruppe aktiviert wird. Im zweiten Schritt wird der aktivierte Katalysator mit einer Mischung aus einer Glykose, Alkalimetallhypophosphit und einem Kohlenhydratpartialester in Kontakt gebracht, wobei letzterer als Emulgator wirkt. Erfindungsgemäß kann das Alkalimetallhypophosphit auch bereits in Schritt (a) zugegeben werden. Diese Verfahrensweise ist im Sinne der Erfindung als äquivalent anzusehen. Während der Reaktion findet eine Übertragung von Acylgruppen auf den Emulgator statt, der in der weiteren Folge der Reaktion selbst als Acylierungsmittel fungiert und Acylgruppen auf die Glykose überträgt, die ihrerseits damit in einen Kohlenhydratpartialester überführt wird. Die Reaktion findet in Abwesenheit von Lösungsmitteln statt, was sowohl aus ökonomischer Sicht als auch im Hinblick auf eine Verwendung im Lebensmittel- oder Kosmetiksektor von erheblichem Vorteil ist. Ein weiterer unerwarteter Vorteil des Verfahrens besteht darin, dass man trotz Reduktion des Alkalimetall-Katalysators eine effektive Umsetzung in vergleichbar kurzen Reaktionszeiten mit verbesserter Farbqualität erzielt.

### Katalysatoren

Als Katalysatoren wird erfindungsgemäß eine Kombination aus Alkalimetallcarbonate(n) und Alkalimetallhypophosphite(en) benutzt. Vorzugsweise wird Natrium- und/oder Kaliumcarbonat eingesetzt sowie Natrium- und/oder Kaliumhypophosphit. Erfindungsgemäß vorteilhaft ist es, 0,06 - 0,6 Mol Alkalimetallcarbonat und 0,01 - 0,1 Mol Alkalimetallhypophosphit pro Mol Glykose einzusetzten. Als erfindungsgemäß besonders vorteilhaft haben sich Einsatzmengen von 0,07 - 0,3 Mol Kaliumcarbonat und 0,01 - 0,05 Mol Natriumhypophosphit pro Mol Glykose, insbesondere 0,08 - 0,2 Mol Kaliumcarbonat und 0,01 - 0,03 Mol Natriumhypophosphit pro Mol Glykose, und ganz besonders bevorzugt 0,1 - 0,15 Mol Kaliumcarbonat und 0,012 - 0,02 Mol Natriumhypophosphit pro Mol Glykose erwiesen.

### Fettsäureniedrigalkylester

Typische Beispiele für geeignete Acylierungsmittel sind die Ester von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen mit Methanol, Ethanol, Propanol, Isopropylalkohol, n-Butanol, i-Butanol, tert.Butanol, n-Pentanol und Isopentanol. Vorzugsweise werden Ester der Formel (I) eingesetzt, in der R² für einen Methyl- und/oder Ethylrest steht. In einer weiteren bevorzugten Variante werden Fettsäurealkylester (a2) mit linearen, gesättigten Acylresten mit 12 bis 30 C-Atomen, vorzugsweise mit 16 bis 24 C-Atomen eingesetzt werden. Besonders bevorzugt sind C16-C24-Fettsäuremethylester, insbesondere C16-C20-Fettsäuremethylester, wobei lineare und unverzweigte Ester unter diesen erfindungsgemäß als besonders bevorzugt gelten. Erfindungsgemäß vorteilhaft ist der Einsatz von C16-Fettsäuremethylestern oder C18-Fettsäuremethylestern oder von beliebigen Gemischen von C16/C18-Fettsäuremethylestern, beispielsweise mit einem C16/C18-Anteil von 50:50 oder 30:70 oder 70:30.

Das molare Einsatzverhältnis von Fettsäurealkylester (a2) : Glykose (b1) beträgt vorzugsweise wenigstens 0,5, insbesondere 0,5 - 2,5 und besonders bevorzugt 0,6 - 2,0. Bei geringeren Molverhältnissen sind die Reaktionsprodukte zunehmend gefärbt und viskos, was vermutlich auf eine Karamelisierung des Zuckers zurückzuführen ist. Erfindungsgemäß besonders bevorzugt ist ein Molverhältnis von 1,3 - 1,6, insbesondere 1,4 - 1,55 um eine Karamelisierung des Zuckers zu reduzieren und farbliche hellere Rohprodukte zu erhalten.

### Herstellung des Katalysatorsystems

Zur Herstellung des Katalysatorsystems, d.h. zur Aktivierung der Fettsäurealkylester als Acylierungsmittel, werden Fettsäurealkylester und Alkalicarbonat unter starkem Rühren vermischt. Hierbei hat es sich erfindungsgemäß als vorteilhaft erwiesen, das katalytisch aktive System bei Temperaturen im Bereich von 50°C bis 100°C, vorzugsweise 60 bis 90°C herzustellen. Insbesondere soll die Temperatur im Falle fester Fettsäurealkylester oberhalb des Schmelzpunktes der Ester liegen, um eine möglichst homogene Dispersion zu ermöglichen. Hierbei findet vermutlich eine Chemisorption des Esters auf der Oberfläche des Carbonats statt. Zur Herstellung einer feinteiligen Dispersion wird das System üblicherweise im oben genannten Temperaturbereich für ca. 15 - 60 Minuten, vorzugsweise 15 - 30 Minuten unter stetigem Rühren gehalten. Im großtechnischen Maßstab ist hierfür beispielsweise der Sigmarührer® SIR der Firma Stelzer mit insgesamt 5 Impellern auf der Rührwelle geeignet.

### Glykolen

Unter den Glykosen werden die auch als Kohlenhydrate bezeichneten Polyhydroxyaldehyde (Aldosen) und Polyhydroxyketone (Ketosen) sowie höhermolekulare Verbindungen zusammengefaßt, die sich durch Hydrolyse in solche Stoffe überführen lassen. Im Sinne der Erfindung können als Glykosen sowohl die monomeren Polyhydroxyaldehyde oder Polyhydroxyketone (Monosaccharide) oder ihre Dimeren bis Decameren (Disaccharide, Trisaccharide, Oligosaccharide) eingesetzt werden. Als Monosaccharide (auch "einfache Zucker" genannt) kommen beispielsweise Biosen, Triosen, Tetraosen, Pentosen, Hexosen, Heptosen etc. in Frage. Typische Beispiele für Aldopentosen sind D-Ribose, D-Xylose und L-Arabinose. Zu den wichtigsten Aldohexosen gehören D-Glucose, D-Mannose und D-Galactose; bei den Ketohexosen sind D-Fructose und Sorbose zu nennen. Die 6-Desoxyzucker L-Fucose und L-Rhamnose sind ebenfalls weit verbreitete Hexosen und kommen als Ausgangsstoffe ebenfalls in Frage. Die einfachsten Oligosaccharide, die als Ausgangsstoffe geeignet sind, stellen die Disaccharide dar. Vorzugsweise werden Saccharose (Rohrzucker, Rübenzucker), Lactose (Milchzucker) und/oder Maltose (Malzzucker) eingesetzt. Der Einsatz von Mono- und/oder Disacchariden ist im Sinne des Verfahrens bevorzugt; insbesondere werden Saccharose oder Glucose bevorzugt eingesetzt.

### Emulgatoren

Im Sinne der Erfindung hat es sich als besonders vorteilhaft erwiesen als Emulgatoren Partialester von Kohlenhydraten einzusetzen, deren Kohlenhydrateinheit mit denen der Zielprodukte identisch ist. Erfindungsgemäß besonders vorteilhaft ist es, Kohlenhydratpartialester einzusetzen, in denen sowohl die Kohlenhydrateinheit als auch der Esterrest mit denen der Zielprodukte übereinstimmt, die sich also ggf. lediglich durch den Veresterungsgrad unterscheiden. Besonders bevorzugt ist der Einsatz von Saccharosepartialestern mit einem durchschnittlichen Veresterungsgrad im Bereich von 2 bis 6, insbesondere von 3 bis 4. Geeignete Zuckerester sind beispielsweise Sisterna® SP 01, Sisterna® SP 30 und Sisterna® SP 50. Erfindungsgemäß bevorzugt als Emulgatoren sind Zuckerester mir einem geringen Monoesteranteil, vorzugsweise weniger als 30 Gew.-% Monoesteranteil und insbesondere weniger als 1 Gew.-% Monoesteranteil. Erfindungsgemäß besonders bevorzugt ist der Einsatz von Partialester der Saccharose mit C16/C18-Fettsäuren, die einen entsprechend niedrigen Monoesterabteil aufweisen, da diese die Reaktionszeit herabsetzen und zu einer schnellen Umsetzung beitragen. Die Kohlenhydratpartialester können als Pulver, in flüssiger Form, aber auch in pelletierter Form eingesetzt werden. So hat es sich beispielsweise als vorteilhaft erwiesen, Pellets aus Saccharosepartialestern mit solchen Fettsäuremethylestern einzusetzen, die auch als Reaktanden verwendet werden.

Als zusätzliche Co-Emulgatoren kommen z.B. nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxid-Anlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglyce-rinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß und/oder Mischester von Fettsäuren mit 6 bis 22 Kohlenstoffatomen, Methylglucose und Polyolen, vorzugsweise Glycerin sowie
(13) Polyalkylenglycole.

Das molare Einsatzverhältnis von Kohlenhydratpartialester (b2) : Glykose (b1) beträgt vorzugsweise 0,03 - 0,25, vorzugsweise 0,04 - 0,2 und besonders bevorzugt 0,09 - 0,12.

Erfindungsgemäß bevorzugt ist ein Verfahren, bei dem das Molverhältnis von (a1) : (a2) : (b1) : (b2) : (b3) im Bereich (0.06 - 0.6) : (0,6 - 2,0) : 1 : (0.04 - 0.2) : (0. 01 - 0.1 ) variiert.

### Reaktionsführunp

Die Reaktion wird dergestalt durchgeführt, dass unter starkem Rühren eine Emulsion/Dispersion hergestellt wird, in welcher das Katalysatorsystem, die Glykose und der Kohlenhydratester sowie gegebenenfalls weitere Emulgatoren enthalten sind. Das mechanische Rühren wird im Falle mittel- bis hochviskoser Gemische im industriellen Maßstab mit einem Rührsystem aus Impellerrührer in Kombination mit Stromstörer bei Umdrehungszahlen von 50 bis 400 Umdrehungen pro Minute, vorzugsweise 100-300 Umdrehungen pro Minute durchgeführt.

Vorzugsweise beträgt der Druck in Schritt (b) und Schritt (c) höchstens 25 mbar und ganz besonders bevorzugt höchstens 15 mbar, insbesondere höchstens 10 mbar. Durch diese Druckbedingungen wird sichergestellt, dass dem Reaktionsgleichgewicht möglichst effizient Wasser entzogen wird. Insbesondere soll die "Vortrocknung" in Schritt (b) dazu dienen, das rohstoffbedingte Restwasser zu entziehen. Schritt (b) wird vorzugsweise bei Temperaturen von 70 - 85 °C, insbesondere 75 - 80 °C und bei einem Druck von 1 bis 25 mbar, vorzugsweise 1 bis 15 mbar durchgeführt. Die eigentliche Veresterungsreaktion, Schritt (c), wird vorzugsweise bei Temperaturen von 100 °C - 125 °C, insbesondere 110 °C - 120 °C, besonders bevorzugt 115 °C- 120 °C und bei einem Druck von 1 bis 25 mbar, vorzugsweise 1 bis 15 mbar und besonders bevorzugt 1 - 10 mbar durchgeführt. Die Veresterungsreaktion (c) wird solange fortgesetzt, bis der Gehalt an Fettsäurealkylestern der Formel (I) auf mindestens 8 Gew-% bezogen auf die Gesamtzusammensetzung, vorzugsweise mindestens 5 Gew.-% bezogen auf die Gesamtzusammensetzung abgefallen ist. Die Reaktionszeiten liegen üblicherweise im Bereich von 5 bis 15 h und können durch effizientes Rühren erheblich reduziert werden. Die so hergestellten Produkte weisen üblicherweise folgende Verteilung auf: 5 - 20 Gew.-% Monoester, 15 - 30 Gew.-% Diester, 20 - 40 Gew.-% Triester und 30 - 40 Gew.-% Tetraester und einen geringen Anteil höherer Homologer. Der Anteil nicht umgesetzter Glykosen im Rohprodukt liegt üblicherweise bei höchsten 15 Gew.-%, vorzugsweise bei höchsten 10 Gew.-%.

In einer weiteren bevorzugten Ausführungsform wird ein leichter Stickstoff-Schutzgasstrom durch die Reaktionsmischung geführt, ohne jedoch die oben angegebenen Druckbedingungen zu verlassen. Diese Verfahrensweise führt zu farblich helleren Produkten.

### Aufarbeitung des Rohgemisches

In einer erfindungsgemäß bevorzugten Verfahrensvariante wird das heiße Reaktionsprodukt in einem für kosmetische Anwendungen und/oder für Lebensmittel geeigneten Emollient gelöst und nach Abtrennung der nicht umgesetzten Glykosen, mit Wasserstoffperoxid - vorzugsweise unter Stickstoffatmosphäre - gebleicht, gegebenenfalls zur Einstellung eines pH-Wertes zwischen 6 und 8 mit einer Säure behandelt wird und dann optional über ein Filtrationshilfsmittel filtriert wird. Unter Emollient im Sinne der Erfindung sind dabei Ölkörper zu verstehen, die bei 60 °C, vorzugsweise schon bei 40 °C und insbesondere schon bei 20 °C und Normaldruck flüssig sind. Die Emollients müssen die nach dem erfindungsgemäßen Verfahren hergestellten Kohlenhydratpartialester lösen können, entweder bei Raumtemperatur oder ggf. in der Wärme. Als Emollient geeignet sind auch Ölkörper, die bei Raumtemperatur fest, pastös oder wachsartig, aber im geschmolzenen Zustand ein gutes Lösungsvermögen für die Kohlenhydratpartialester haben. bei das Erfindungsgemäß geeignet sind beispielsweise Kohlenwasserstoffe, Esteröle, Polyole, Dialkylether, Dialkylcarbonate, wie beispielsweise Cetiol® S, Sylko® 365 NF, Panalane® L 14 E, Cetiol® NPC, Cetiol® SN, Cetiol® PGL, Edenor® V, Cetiol® OE, Cetiol® CC. Erfindungsgemäß bevorzugt geeignet sind Kohlenwasserstoffe und unter diesen ein bei 20°C und Normaldruck flüssiges Polyisobuten, insbesondere ein gehärtetes Polyisobuten, das unter der Bezeichnung Panalane® L14 E (Hersteller: Amoco; INCI-Bezeichnung: hydrogenated polyisobutene) im Handel ist. Letzteres zeichnet sich neben einer niedrigen Viskosität durch sehr gute Lösungseigenschaften für die erfindungsgemäß hergestellten Sucroseester und sensorische Vorteile bezüglich der kosmetischen Endformulierungen aus. Auch flüssige, pastöse oder wachsartige Esteröle aus C6-C22-Fettsäuren und C1-C3-Alkoholen wie z.B. Edenor® ME 16V sind als Lösungsmittel für erfindungsgemäß hergestellten Kohlenhydratpartialester gut geeignet.

Die Abtrennung der nicht umgesetzten Glykosen kann beispielsweise durch Dekantieren, Zentrifugieren oder durch Filtration erfolgen. Erfindungsgemäß bevorzugt ist es, die Abtrennung durch Filtration vorzunehmen, vorzugsweise mit beheizbaren Filtern. Vorzugsweise wird die Filtration bei 60 - 80°C vorgenommen.

Als Filter geeignet sind z.B. Filter auf Basis von Cellulose und Kieselerde wie Fibra Fix® AF6 der Filtrox AG mit einem Durchsatz von ca. 2800 - 3600 l/m² pro Minute (bezogen auf Wasser bei 100 kPa), der für eine Rohfiltration geeignet ist. Geeignete Filtrationshilfsmittel sind beispielsweise Hyflo Super Cel® (Lehmann & Voss), Becolite® 5000 (Begerow Chemie), Arbocel® BC 200 und B600, Filtracel® AFC 1400 (J. Rettenmaier & Söhne GmbH & Co.), TriSyl® oder TriSyl® 2 (Grace Davison), Bleicherden (z.B. Tonsil® von Süd-Chemie) und Kieselerden, z.B. Diatomeenerden wie Seitz Ultra® (Pall Corporation).

Je nach Filtrationshilfsmittel werden trübe bis klare, viskose, mehr oder minder gefärbte Produkte erhalten. Die Produkte sind deutlich heller gefärbt als dies beim alleinigen Einsatz von Alkalimetallcarbonaten der Fall ist. Bezüglich Farbqualität und Durchsatz bei der Filtration haben sich Kieselerden erfindungsgemäß besonders bewährt. Insbesondere ist ein Produkt, das unter dem Namen Seitz Ultra® im Handel ist, erfindungsgemäß bevorzugt geeignet. Für die Filtration wird das Filtrationshilfsmittel zugegeben, das Gemisch unter stetigem Rühren homogenisiert und dann abfiltriert. Nach der ersten Filtration werden üblicherweise Produkte erhalten, deren Restzuckergehalt kleiner 10 Gew.-%, insbesondere kleiner 5 Gew.-% bezogen auf die Gesamtzusammensetzung des Produktgemisches ist. An diesen ersten Filtrationsschritt schließt sich ein Bleichungsschritt mit Wasserstoffperoxid an, der vorzugsweise unter Stickstoffatmosphäre durchgeführt wird. Das Reaktionsprodukt wird dann nochmals unter Vakuum getrocknet, um Restmengen an Wasser zu entfernen. Der pH-Wert des Produktgemisches sollte zwischen 6 und 8 liegen und wird gegebenenfalls durch Zugabe von Säure eingestellt. Hierfür können die üblichen Mineralsäuren oder Fruchtsäuern eingesetzt werden. Erfindungsgemäß besonders geeignet ist die Zugabe von Zitronensäure oder Milchsäure. Milchsäure ist besonders geeignet, da die so neutralisierten Produkte zu stabileren kosmetischen Endformulierungen führen.

Die Filtration wird gegebenenfalls mehrmals wiederholt, um den Restzuckergehalt zu minimieren. Die weiteren Filtrationen können mit Filtern mit einer Permeabilität von ca. 240 - 300 l/m² pro Minute (bezogen auf Wasser bei 100 kPa) durchgeführt werden. Erfindungsgemäß geeignet ist beispielsweise Fibra Fix® AF 41 H der Filtrox AG, ein Filter auf Cellulosebasis und anorganischen Zusatzstoffen. Die Minimierung des Restzuckergehaltes ist vorteilhaft, um Produkte mit guter farblicher Qualität, zu erhalten. Je höher der Restzuckergehalt desto dunkler sind die Produkte und desto mehr neigen sie im Laufe der Zeit zu einer weiteren Dunkelfärbung. Um den Gehalt an freier Fettsäure und Seife im Endprodukt zu minimieren, sollten alle Nachbehandlungsschritte zur Aufarbeitung des Rohgemisches in möglichst kurzer Zeit durchgeführt werden.

In einer bevorzugten Variante des erfindungsgemäßen Verfahrens wird der Lösung des Kohlenhydratpartialesters ein C16-C40-Fettalkohol, vorzugsweise ein C18-C30 und besonders bevorzugt eine C20-C24-Fettalkohol oder ein beliebiges Gemisch dieser Fettalkohole zugegeben, gegebenenfalls mit Wasserstoffperoxid nachgebleicht und das Produkt pelletiert, extrudiert, granuliert, kristallisiert, sprühgetrocknet oder tablettiert. Die Zugabe erfolgt üblicherweise zur heißen Lösung bei ca. 70 - 85°C. Erfindungsgemäß bevorzugt ist es 1 - 10 Gew.-%, vorzugsweise 1 - 5 Gew.-% eines C16-C40-Fettalkohols oder ein beliebigen Gemisches dieser Fettalkohole bezogen auf die resultierende Gesamtzusammensetzung zuzugeben. Erst die Zugabe der Fettalkohole ermöglicht es, Formkörper zu erhalten, die kein Öl (z.B. Kohlenwasserstoff) mehr ausscheiden. Formkörper ohne Fettalkohole scheiden im Laufe der Zeit Öl aus, d.h. sie "schwitzen" und sind daher weniger lagerstabil. Als erfindungemäß vorteilhaft haben sich C20-C24-Fettalkohole erwiesen.

Erfindungsgemäß besonders geeignet ist die Zugabe eines C22-Fettalkohols, Behenylalkohol, der z.B. unter der Bezeichnung Lanette® 22 von der Cognis Deutschland GmbH & Co. KG vermarktet wird, insbesondere in Menge von 1 - 5 Gew.-%, vorzugsweise 1 - 3 Gew.-% bezogen auf die Endformulierung.

Ein weiterer Gegenstand der Erfindung sind Formkörper enthaltend (a) Kohlenhydratpartialester, erhältlich nach einem Verfahren gemäß wenigstens einem der Ansprüche 1 bis 16, (b) ein für kosmetische Anwendungen und/oder für Lebensmittel geeignetes Emollient für die Kohlenhydratpartialester und (c) 1 - 10 Gew.-% eines C16-C40-Fettalkohols oder eines beliebigen Gemisches dieser Fettalkohole, wobei der Gehalt an weiteren Hilfs- und Zusatzstoffen höchstens 20 Gew.-% beträgt. Eine bevorzugte Ausführungsform des Formkörpers enthält (a) 10 - 80 Gew.-% eines Kohlenhydratpartialesters bzw. eines Gemisches aus Kohlenhydratpartialestern erhältlich nach einem Verfahren gemäß wenigstens einem der Ansprüche 1 bis 16, (b) 10 - 20 Gew.-% eines für kosmetische Anwendungen und/oder für Lebensmittel geeigneten Emollients (c) 1 - 10 Gew.-% eines C16-C40-Fettalkohols oder eines beliebigen Gemisches dieser Fettalkohole, wobei der Gehalt an weiteren Hilfs- und Zusatzstoffen höchstens 10 Gew.-% beträgt. Vorzugsweise beträgt der Gehalt an weiteren Hilfs- und Zusatzstoffen weniger als 10 Gew.-%, insbesondere weniger als 5 Gew.-% and der Gesamtzusammensetzung. Ganz besonders bevorzugt beschränkt sich der Anteil an weiteren Hilfs- und Zusatzstoffen lediglich auf Nebenprodukte, die reaktionsbedingt im Produktgemisch enthalten sind. Die Definition für Emollient ist dieselbe wie beim Verfahren (vide supra). Erfindungsgemäß geeignet sind die bereits vorher erwähnten Stoffe, beispielsweise Kohlenwasserstoffe, Esteröle, Polyole, Dialkylether, Dialkylcarbonate, wie Cetiol® S, Sylko® 365 NF, Panalane® L 14 E, Cetiol® NPC, Cetiol® SN, Cetiol® PGL, Edenor® V, Cetiol® OE, Cetiol® CC. Erfindungsgemäß bevorzugt geeignet sind Kohlenwasserstoffe und unter diesen ein bei 20°C und Normaldruck flüssiges Polyisobuten, insbesondere ein gehärtetes Polyisobuten, das unter der Bezeichnung Panalane® L14 E (Hersteller: Amoco; INCI-Bezeichnung: hydrogenated polyisobutene) im Handel ist. Auch flüssige, pastöse oder wachsartige Esteröle aus C6-C22-Fettsäuren und C1-C3-Alkoholen, wie z.B. Edenor® ME 16V sind als Lösungsmittel für erfindungsgemäß hergestellten Kohlenhydratpartialester besonders geeignet. Erfindungsgemäß bevorzugte Fettalkohole sind diejenigen, die auch für das Verfahren genannt wurden (vide supra). Die erfindungsgemäßen Formkörper werden vorzugsweise durch Pelletierung, Extrusion, Granulation, Sprühtrocknung, Kristallisation, Tablettierung oder durch Coaten erhalten. Die Verfahren hierzu sind dem Fachmann hinlänglich bekannt und in der Literatur beschrieben.

### Gewerbliche Anwendbarkeit

Die nach dem erfindungsgemäßen Verfahren erhältlichen Kohlenhydratester weisen ausgezeichnete grenzflächenaktive Eigenschaften auf und können beispielsweise als Emulgatoren für die Herstellung von Lebensmitteln (Brot, Backwaren, Speiseeis etc.) und kosmetischen Präparaten dienen, in denen sie in Mengen von 0,1 bis 20 Gew.-%, vorzugsweise 1 bis 10 Gew.-% und insbesondere 1 bis 5 Gew.-% enthalten sein können. Die erfindungsgemäßen Kohlenhydratester können auch als Emulgatoren zur Herstellung von Polyacryl- bzw. Polymethacrylsäureverbindungen, die als Superadsorber beispielsweise für Babywindeln dienen können, eingesetzt werden. Da die Emulgatoren im Produkt verbleiben, kommen hier nicht nur ihre ausgezeichneten anwendungstechnischen Eigenschaften, sondern auch ihre besondere Hautverträglichkeit zum Tragen.

Ein weiterer Gegenstand der Erfindung ist die Verwendung der nach dem erfindungsgemäßen Verfahren hergestellten Kohlenhydratpartialester als Emulgatoren in Lebensmitteln und kosmetischen oder pharmazeutischen Zubereitungen.

Gegenstand der Anmeldung sind auch kosmetische Mittel, die 0,1 - 20 Gew.-%, vorzugsweise 0,5 - 7 Gew.-%, der erfindungsgemäßen Formkörpers enthalten sowie die Verwendung der Formkörper zur Herstellung kosmetischer Mittel. Diese tragen dazu bei lamellare Strukturen aufzubauen, die den Endformulierungen besondere Stabiltät verleihen.

Gegenstand der Erfindung ist daher auch die Verwendung der erfindungsgemäßen Formkörper zum Aufbau lamellarer Strukturen in O/W-Emulsionen.

Je nach Applikationszweck enthalten die kosmetischen Formulierungen eine Reihe weiterer Hilfs- und Zusatzstoffe wie beispielsweise Verdickungsmittel, Überfettungsmittel, Stabilisatoren, Polymere, Lecithine, Phospholipide, biogene Wirkstoffe, UV-Lichtschutzfaktoren, Antioxidantien, Deodorantien, Filmbildner, Quellmittel, Insektenrepellentien, Hydrotrope, Solubilisatoren, Konservierungsmittel, Parfümöle, Farbstoffe etc. Die Mengen der jeweiligen Zusätze richten sich nach der beabsichtigten Verwendung.

### Beispiele

### Beispiel 1 (Labormaßstab)

Die Synthese wurde unter einem leichten Stickstoffstrom durchgeführt, der in die Reaktionsmischung eingeleitet wurde.

In einem 2 kg-Doppelmantel-Glasreaktor mit schnell laufendem Rührer, Rückflußkühler und Tropftrichter wurden 636,7g (2,2 mol) gehärteter Palmfettsäuremethylester (Edenor® ME AS 16 V) vorgelegt und auf 65°C erwärmt. Bei einer Rührergeschwindigkeit von 1300 UpM wurden 26,7g (0.19 Mol) Kaliumcarbonat, entsprechend 4 Gew.-% bezogen auf den Methylester zugegeben. Das Gemisch wurde 10 Minuten gerührt. Zu der so erhaltenen Dispersion wurde portionsweise 134g (0.16 Mol) Sucrosedistearat (Sisterna® SP 30C) zugegeben, 10 Minuten bei 1500 Upm gerührt, dann 503g (1,47 Mol) Saccharose (Puderzucker von Beguin Say) zugegeben, 20 Minuten bei 2000 UpM gerührt, dann 2 g (0.023 Mol) Natriumhypophoshit zugegeben, wobei die Rührgeschwindigkeit bei 2000 UpM gehalten wurde. Anschließend wurde die resultierende Emulsion/Dispersion bei einem Druck von 1 - 5 mbar auf 85 °C erhitzt und weitere 30 Minuten gerührt. Dann wurde die Temperatur langsam auf 125°C erhöht und die Reaktionsmischung bei einem Druck von 7 mbar und einer Rührergeschwindigkeit von 2000 UpM weitere 11 h gerührt. Es resultierte ein Saccharoseester mit einem Gehalt von 14 Gew.-% Monoester, 22 Gew.-% Diester, 30 Gew.-% Triester and 34 Gew.-% Tetraester und höheren Homologen - bezogen auf die Gesamtmenge Kohlenhydratester. Der Gehalt an nichtabreagiertem Ester in der Reaktionsmischung lag bei ca. 5 Gew.-%, der Gehalt an freier Saccharose bei 4 Gew.-%.

### Aufarbeitung:

Nachdem das Reaktionsgemisch auf ca. 110 °C abgekühlt war, wurden 244 g Panalane L 14 E (Amoco) zugegeben und die Mischung unter Rühren ca. 15 Minuten homogenisiert. Die heiße Lösung wurde über einen Filter unter Verwendung von Seitz Ultra® als Filtrierhilfsmittel abfiltriert und unter Stickstoffatmosphäre 1 Stunde lang mit 15 ml (0.29 Mol) einer 35 %igen (Gew.-%) Wasserstoffperoxidlösung gebleicht. Der pH-Wert wurde durch Zugabe von 7.2 ml einer 80 %igen Milchsäurelösung (Gew.-%) eingestellt und die Lösung einer weiteren Filtration mit Seitz Ultra® unterworfen. Bei 80 °C wurden 2 Gew.-% (bezogen auf die Gesamtzusammensetzung) Behenylalkohol zugegeben, unter Rühren homogenisiert und das Produkt pelletiert.

### Beispiel 2 (Industriemaßstab)

Die Reaktion wurde unter Anspassung an den großindustriellen Maßstab (z.B. entsprechendes Rührwerk) analog zu Beispiel 1 durchgeführt.

Es wurden 887 kg Palmfettsäuremethylester, 37 kg Katalysatormischung (Kaliumcarbonat und Natriumhypophosphit), 187 kg Sucrosedistearat und 700 kg Saccharose miteinander umgesetzt. Es resultierte ein Saccharoseester mit einem Gehalt von 12,3 Gew.-% Monoester, 25,1 Gew.-% Diester, 33,1 Gew.-% Triester, 31,5 Gew.-% Tetraster und höheren Homologen - bezogen auf die Gesamtmenge Kohlenhydratester. Der Gehalt an nichtabreagiertem Ester in der Reaktionsmischung lag bei ca. 6 Gew.-%. Die Aufarbeitung erfolgte analog zu Beispiel 1.

### Aufarbeitung

Das nach Beispiel 2 erhaltene Produkt wurde statt mit Panalane L 14 E mit einem gehärteten Palmfettsäuremethylester (Edenor® ME AS 16 V) aufgearbeitet.

Nachdem das Reaktionsgemisch auf ca. 100°C°C abgekühlt war, wurden 320 kg Palmfettsäuremethylester (Edenor® ME AS 16 V; Cognis) zugegeben und die Mischung unter Rühren ca. 45 Minuten homogenisiert. Die heiße Lösung wurde über einen Filter unter Verwendung von Seitz Ultra® als Filtrierhilfsmittel abfiltriert und unter Stickstoffatmosphäre 45 Minuten lang mit 10Kg einer 35% igen (Gew.-%) Wasserstoffperoxidlösung bei 85°C gebleicht. Der pH-Wert wurde durch Zugabe von 9 kg einer 80%-igen Milchsäurelösung (Gew.-%) eingestellt und die Lösung einer weiteren Filtration mit Seitz Ultra® unterworfen.

**Tabelle 1: Einfluss der Menge des Katalysators Kaliumcarbonat (Vergleich) auf den Umsatz des Fettsäuremethylesters**

| Die Umsetzung wurde analog zu Beispiel 1 durchgeführt. Als Fettsäuremethylester (ME) wurde ein C16/C18-Fettsäuremethylester mit einem Anteil von C16:C18-Fettsäure von 50:50 eingesetzt. Als Kohlenhydratpartialester (SE) wurde Sisterna® SP 30C eingesetzt. Die Gew.-% Katalysator (K₂CO₃) beziehen sich auf den Gehalt in der Gesamtzusammensetzung. Die Angabe des Umsatzes in % bezieht sich auf Gew.-% des Fettsäuremethylesters in der Gesamtreaktionsmischung. | | | |
|---|---|---|---|
| **Zeit** | **K₂CO₃ 5 Gew.-% V1** | **K₂CO₃ 2.5 Gew.-% V2** | **K₂CO₃ 1.5 Gew.% V3** |
| | Umsatz % ME | Umsatz % ME | Umsatz % ME |
| 3h | 31,2 | 30,9 | 41,1 |
| 4h | 27,5 | 27,3 | |
| 5h | 22,0 | 28,9 | 31,4 |
| 6h | 19,1 | | |
| 7h | 15,7 | 24,8 | 23,8 |
| 8h | 10,7 | | |
| 9h | | 10,2 | 19,5 |
| 10h | | 7,1 | |
| 11h | | | 13,0 |
| **ME** | **475 g** | **487.5 g** | **640.2 g** |
| **SE** | **100 g** | **102.6 g** | **134.8 g** |
| **K₂CO₃** | **50 g** | **25 g** | **19.5 g** |
| **Saccharose** | **375 g** | **384.9 g** | **505.4 g** |

**Tabelle 2: Vergleich zwischen erfindungsgemäßem Katalysatorgemisch (B1) und Alkalimetallcarbonaten (V4 und V5)**

| Die Umsetzung wurde analog zu Beispiel 1 durchgeführt. Als Fettsäuremethylester (ME) wurde ein C16/C18-Fettsäuremethylester mit einem Anteil von C16:C18-Fettsäure von 50:50 eingesetzt. Als Kohlenhydratpartialester (SE) wurde Sisterna® SP 30C eingesetzt. Die Gew.-% Katalysator (K₂CO₃ in V4; K₂CO₃ und NaH₂PO₂ in B1 sowie Na₂CO₃ in V5) beziehen sich auf den Gehalt in der Gesamtzusammensetzung. Die Angabe des Umsatzes in % bezieht sich auf Gew.-% des Fettsäuremethylesters in der Gesamtreaktionsmischung. | | | |
|---|---|---|---|
| **Zeit** | **K₂CO₃ 2.5 Gew.-% V4** | **2.5 Gew.-% K₂CO₃ 0.2 Gew.-% NaH₂PO₂ B1** | **1.92 Gew.-% Na₂CO₃ V5** |
| | Umsatz % ME | Umsatz % ME | Umsatz % ME |
| 3h | 30,9 | 28,4 | 42,2 |
| 4h | 27,3 | | |
| 5h | 28,9 | 19,7 | 38,1 |
| 6h | | | |
| 7h | 24,8 | 13,3 | Keine Veränderung mehr |
| 8h | | | Keine Veränderung mehr |
| 9h | 10,2 | | Keine Veränderung mehr |
| 10h | 7,1 | 3,9 | Keine Veränderung mehr |
| | | | |
| **ME** | **487.5** | **634.3** | **637.5** |
| **SE** | **102.6** | **133.4** | **134.2** |
| **K₂CO₃** | **2.5** | **32.5** | **-** |
| **NaH₂PO₂** | **-** | **2.6** | **-** |
| **Saccharose** | **384.5** | **500.4** | **503.4** |
| **Na₂CO₃** | **-** | **-** | **25** |

**Tabelle 3: Erfindungsgemäße Katalysatorzusammensetzungen und prozentualer Umsatz des C16/18-Fettsäuremethylesters**

| Die Umsetzung wurde analog zu Beispiel 1 durchgeführt. Als Fettsäuremethylester (ME) wurde ein C16/C18-Fettsäuremethylester mit einem Anteil von C16:C18-Fettsäure von 50:50 eingesetzt. Als Kohlenhydratpartialester (SE) wurde Sisterna® SP 30C eingesetzt. Die Gew.-% des erfindungsgemäßen Katalysatorgemisches beziehen sich auf den Gehalt in der Gesamtzusammensetzung. Die Angabe des Umsatzes in % bezieht sich auf Gew.-% des Fettsäuremethylesters in der Gesamtreaktionsmischung. | | | |
|---|---|---|---|
| **Zeit** | **2.5 Gew.-% K₂CO₃ 0.2% Gew.-% NaH₂PO₂ B2** | **1.9 Gew.-% K₂CO₃ 0.1% Gew.-% NaH₂PO₂ B3** | **1.5 Gew.-% K₂CO₃ 0.5% Gew.-% NaH₂PO₂ B4** |
| | Umsatz % ME | Umsatz % ME | Umsatz % ME |
| 3h | 28,4 | | 24,5 |
| 4h | | | |
| 5h | 19,7 | | 21,5 |
| 6h | | | |
| 7h | 13,3 | 9,2 | 15,6 |
| 8h | | | |
| 9h | | 4,8 | |
| 10h | 3,9 | | 6,3 |
| 11h | | 3,6 | |
| | | | |
| **ME** | **634.3** | **636.7** | **637** |
| **SE** | **133.4** | **134** | **134.2** |
| **K₂CO₃** | **32.5** | **24.7** | **19.2** |
| **NaH₂PO₂** | **2.6** | **2** | **6.5** |
| **Saccharose** | **500.4** | **502.6** | **502.8** |

1. Solvensfreies Verfahren zur Herstellung von Kohlenhydratpartialestern mit einem durchschnittlichen Veresterungsgrad im Bereich von 1 bis 4 durch alkalikatalysierte Veresterung in Gegenwart eines Katalysator-Gemisches aus Alkalimetallcarbonat und Alkalimetallhypophosphit, wobei man
   (a) zur Bildung eines katalytisch aktiven Systems wenigstens ein Alkalimetallcarbonat (a1) und wenigstens ein Fettsäurealkylester (a2) der Formel

      R¹CO-OR² (I)

      mischt, in der R¹CO für einen linearen oder verzweigten, gesättigten oder ungesättigten Acylrest mit 6 bis 30 Kohlenstoffatomen und R² für einen linearen oder verzweigten Alkylrest mit 1 bis 5 Kohlenstoffatomen steht, und
   (b) zu der aus (a) resultierenden Mischung unter stetigem Rühren (b1) Glykosen mit 5 bis 12 Kohlenstoffatomen, (b2) Kohlenhydratpartialestern als Emulgatoren und (b3) Alkalimetallhypophosphit zugibt, so daß eine Dispersion resultiert, und dem resultierenden Gemisch unter stetigem Rühren bei Temperaturen bis zu 100° C bei einem Druck von bis zu 50 mbar Wasser entzieht und
   (c) die Veresterungsreaktion bei einem Druck von bis zu 50 mbar und Temperaturen bis zu 125°C unter stetigem Rühren solange fortsetzt, bis der Gehalt an Fettsäurealkylestern der Formel (I) auf mindestens 8 Gew-% bezogen auf die Gesamtzusammensetzung abgefallen ist.
   wobei die Schritte (a) bis (c) optional unter einer Schutzgasatmosphäre durchgeführt werden.
2. Verfahren nach Anspruch 1, **dadurch gekennzeichnet, dass** man als Alkalimetallcarbonate Natrium- und/oder Kaliumcarbonat einsetzt.
3. Verfahren nach wenigstens einem der Ansprüche 1 bis 2, **dadurch gekennzeichnet, dass** man als Alkalimetallhypophosphit Natrium- und/oder Kaliumhypophosphit einsetzt.
4. Verfahren nach wenigstens einem der Ansprüche 1 bis 3, **dadurch gekennzeichnet, dass** man Ester der Formel (I) einsetzt, in der R² für einen Methyl- und/oder Ethylrest steht.
5. Verfahren nach wenigstens einem der Ansprüche 1 bis 4, **dadurch gekennzeichnet, dass** man das katalytisch aktive System gemäß (a) bei Temperaturen von 50 bis 100°C herstellt.
6. Verfahren nach wenigstens einem der Ansprüche 1 bis 5, **dadurch gekennzeichnet, dass** man als Glykosen Mono- und/oder Disaccharide einsetzt.
7. Verfahren nach wenigstens einem der Ansprüche 1 bis 6, **dadurch gekennzeichnet, dass** man als Emulgatoren Partialester von Kohlenhydraten einsetzt, deren Kohlenhydrateinheit mit denen der Zielprodukte identisch ist.
8. Verfahren nach wenigstens einem der Ansprüche 1 bis 7, **dadurch gekennzeichnet, dass** man als Emulgatoren Saccharosepartialester mit einem durchschnittlichen Veresterungsgrad im Bereich von 2 bis 6, vorzugsweise 3 bis 4 einsetzt.
9. Verfahren nach wenigstens einem der Ansprüche 1 bis 8, **dadurch gekennzeichnet, dass** man die Veresterung gemäß (c) bei Temperaturen im Bereich von 100 bis 125°C und bei einem Druck im Bereich von 1 bis 25 mbar durchführt.
10. Verfahren nach wenigstens einem der Ansprüche 1 bis 9, **dadurch gekennzeichnet, dass** man als Schutzgas Stickstoff einsetzt.
11. Verfahren nach wenigstens einem der Ansprüche 1 bis 10, **dadurch gekennzeichnet, dass** man 0,06 - 0,6 Mol Alkalimetallcarbonat und 0,01 - 0,1 Mol Alkalimetallhypophosphit pro Mol Glykose einsetzt.
12. Verfahren nach wenigstens einem der Ansprüche 1 bis 11, **dadurch gekennzeichnet, dass** das Molverhältnis von Fettsäurealkylester (a2) zu Glykose (b1) wenigstens 0,5 beträgt.
13. Verfahren nach wenigstens einem der Ansprüche 1 bis 11, **dadurch gekennzeichnet, dass** das Molverhältnis (a1) : (a2) : (b1) : (b2) : (b3) im Bereich (0.06 - 0.6) : (0,6 - 2,0) : 1 : (0.04 - 0.2) : (0. 01 - 0.1) variiert.
14. Verfahren nach wenigstens einem der Ansprüche 1 bis 12, **dadurch gekennzeichnet, dass** das heiße Reaktionsprodukt in einem für kosmetische Anwendungen und/oder für Lebensmittel geeigneten Emollient gelöst wird und nach Abtrennung der nicht umgesetzten Glykosen, mit Wasserstoffperoxid, vorzugsweise unter Stickstoffatmosphäre, gebleicht wird, gegebenenfalls zur Einstellung eines pH-Wertes zwischen 6 und 8 mit einer Säure behandelt wird und dann optional über ein Filtrationshilfsmittel filtriert wird.
15. Verfahren nach Anspruch 14, **dadurch gekennzeichnet, dass** das Emollient ein Kohlenwasserstoff, vorzugsweise ein bei 20°C flüssiges Polyisobuten ist.
16. Verfahren nach wenigstens einem der Ansprüche 1 bis 15, **dadurch gekennzeichnet, dass** Fettsäurealkylester (a2) mit linearen, gesättigten Acylresten mit 12 bis 30 C-Atomen, vorzugsweise mit 16 bis 24 C-Atomen eingesetzt werden.
17. Verfahren nach wenigstens einem der Ansprüche 14 bis 17, **dadurch gekennzeichnet, dass** der Lösung des Kohlenhydratpartialesters ein C16-C40-Fettalkohol, vorzugsweise ein C18-C30 und besonders bevorzugt eine C20 - C24 Fettalkohol oder ein beliebiges Gemisch dieser Fettalkohole zugegeben wird, gegebenenfalls mit Wasserstoffperoxid nachgebleicht wird und das Produkt pelletiert, extrudiert, granuliert, kristallisiert, sprühgetrocknet oder tablettiert wird.
18. Verfahren nach Anspruch 17, **dadurch gekennzeichnet, dass** 1 - 10 Gew.-%, vorzugsweise 1 - 5 Gew.-% eines C16-C40-Fettalkohols oder ein beliebigen Gemisches dieser Fettalkohole bezogen auf die resultierende Gesamtzusammensetzung zugegeben wird.
19. Formkörper enthaltend
   (a) Kohlenhydratpartialester, erhältlich nach einem Verfahren gemäß wenigstens einem der Ansprüche 1 bis 16,
   (b) ein für kosmetische Anwendungen und/oder für Lebensmittel geeignetes Emollient und
   (c) 1 - 10 Gew.-% eines C16-C40-Fettalkohols oder eines beliebigen Gemisches dieser Fettalkohole,
   wobei der Gehalt an weiteren Hilfs- und Zusatzstoffen höchstens 20 Gew.-% beträgt.
20. Formkörper gemäß Anspruch 19 enthaltend
   (a) 10 - 80 Gew.-% eines Kohlenhydratpartialesters bzw. eines Gemisches aus Kohlenhydratpartialestern erhältlich nach einem Verfahren gemäß wenigstens einem der Ansprüche 1 bis 16,
   (b) 10 - 20 Gew.-% eines für kosmetische Anwendungen und/oder für Lebensmittel geeigneten Emollients und
   (c) 1 - 10 Gew.-% eines C16-C40-Fettalkohols oder eines beliebigen Gemisches dieser Fettalkohole,
   wobei der Gehalt an weiteren Hilfs- und Zusatzstoffen höchstens 10 Gew.-% beträgt.
21. Formkörper gemäß Anspruch 19, **dadurch gekennzeichnet, dass** er durch Pelletierung, Extrusion, Granulation, Sprühtrocknung, Kristallisation, Tablettierung oder durch Coaten hergestellt wird.
22. Verwendung von Kohlenhydratpartialestern hergestellt nach einem Verfahren gemäß wenigstens einem der Ansprüche 1 bis 16 als Emulgatoren in Lebensmitteln und kosmetischen oder pharmazeutischen Zubereitungen.
23. Kosmetische Mittel, enthaltend einen 0,1 - 20 Gew.-% eines Formkörpers gemäß einem der Ansprüche 19 bis 21.
24. Verwendung der Formkörper gemäß Anspruch 19 bis 21 zur Herstellung kosmetischer Mittel.
25. Verwendung der Formkörper gemäß Anspruch 19 bis 21 zum Aufbau lamellarer Strukturen in O/W-Emulsionen.

## Patentansprüche

1. Formkörper enthaltend
a. Kohlenhydratpartialester mit einem durchschnittlichen Veresterungsgrad im Bereich von 1 bis 4 erhältlich nach einem solvensfreien Verfahren durch alkalikatalysierte Veresterung in Gegenwart eines Katalysator-Gemisches aus Alkalimetallcarbonat und Alkalimetallhypophosphit, wobei man entweder
(a) zur Bildung eines katalytisch aktiven Systems wenigstens ein Alkalimetallcarbonat (a1) und wenigstens ein Fettsäurealkylester (a2) der Formel
R¹CO-OR² (I)
mischt, in der R¹CO für einen linearen oder verzweigten, gesättigten oder ungesättigten Acylrest mit 6 bis 30 Kohlenstoffatomen und R² für einen linearen oder verzweigten Alkylrest mit 1 bis 5 Kohlenstoffatomen steht, und
(b) zu der aus (a) resultierenden Mischung unter stetigem Rühren (b1) Glykosen mit 5 bis 12 Kohlenstoffatomen, (b2) Kohlenhydratpartialestern als Emulgatoren und (b3) Alkalimetallhypophosphit zugibt, so dass eine Dispersion resultiert, und dem resultierenden Gemisch unter stetigem Rühren bei Temperaturen bis zu 100° C bei einem Druck von bis zu 50 mbar Wasser entzieht und
(c) die Veresterungsreaktion bei einem Druck von bis zu 50 mbar und Temperaturen bis zu 125°C unter stetigem Rühren solange fortsetzt, bis der Gehalt an Fettsäurealkylestern der Formel (I) auf mindestens 8 Gew-% bezogen auf die Gesamtzusammensetzung abgefallen ist.
wobei die Schritte (a) bis (c) optional unter einer Schutzgasatmosphäre durchgeführt werden, oder das Alkalimetallhypophosphit bereits im Schritt (a) zugibt.
b. ein für kosmetische Anwendungen und/oder für Lebensmittel geeignetes Emollient und
c. 1 - 10 Gew.-% eines C16-C40-Fettalkohols oder eines beliebigen Gemisches dieser Fettalkohole,
wobei der Gehalt an weiteren Hilfs- und Zusatzstoffen höchstens 20 Gew.-% beträgt.

2. Formkörper gemäß Anspruch 1 enthaltend
a. 10 - 80 Gew.-% eines Kohlenhydratpartialesters bzw. eines Gemisches aus Kohlenhydratpartialestern
b. 10 - 20 Gew.-% eines für kosmetische Anwendungen und/oder für Lebensmittel geeigneten Emollients und
c. 1 - 10 Gew.-% eines C16-C40-Fettalkohols oder eines beliebigen Gemisches dieser Fettalkohole,
wobei der Gehalt an weiteren Hilfs- und Zusatzstoffen höchstens 10 Gew.-% beträgt.

3. Formkörper gemäß Anspruch 1, **dadurch gekennzeichnet, dass** er durch Pelletierung, Extrusion, Granulation, Sprühtrocknung, Kristallisation, Tablettierung oder durch Coaten hergestellt wird.

4. Verwendung von Kohlenhydratpartialestern mit einem durchschnittlichen Veresterungsgrad im Bereich von 1 bis 4 hergestellt nach einem solvensfreien Verfahren durch alkalikatalysierte Veresterung in Gegenwart eines Katalysator-Gemisches aus Alkalimetallcarbonat und Alkalimetallhypophosphit, wobei man entweder
(a) zur Bildung eines katalytisch aktiven Systems wenigstens ein Alkalimetallcarbonat (a1) und wenigstens ein Fettsäurealkylester (a2) der Formel
R¹CO-OR² (I)
mischt, in der R¹CO für einen linearen oder verzweigten, gesättigten oder ungesättigten Acylrest mit 6 bis 30 Kohlenstoffatomen und R² für einen linearen oder verzweigten Alkylrest mit 1 bis 5 Kohlenstoffatomen steht, und
(b) zu der aus (a) resultierenden Mischung unter stetigem Rühren (b1) Glykosen mit 5 bis 12 Kohlenstoffatomen, (b2) Kohlenhydratpartialestern als Emulgatoren und (b3) Alkalimetallhypophosphit zugibt, so daß eine Dispersion resultiert, und dem resultierenden Gemisch unter stetigem Rühren bei Temperaturen bis zu 100° C bei einem Druck von bis zu 50 mbar Wasser entzieht und
(c) die Veresterungsreaktion bei einem Druck von bis zu 50 mbar und Temperaturen bis zu 125°C unter stetigem Rühren solange fortsetzt, bis der Gehalt an Fettsäurealkylestern der Formel (I) auf mindestens 8 Gew-% bezogen auf die Gesamtzusammensetzung abgefallen ist.
wobei die Schritte (a) bis (c) optional unter einer Schutzgasatmosphäre durchgeführt werden.
oder das Alkalimetallhypophosphit bereits im Schritt (a) zugibt
als Emulgatoren in Lebensmitteln und kosmetischen oder pharmazeutischen Zubereitungen.

5. Kosmetische Mittel, enthaltend einen 0,1 - 20 Gew.-% eines Formkörpers gemäß einem der Ansprüche 1 bis 3.

6. Verwendung der Formkörper gemäß Anspruch 1 bis 3 zur Herstellung kosmetischer Mittel.

7. Verwendung der Formkörper gemäß Anspruch 1 bis 3 zum Aufbau lamellarer Strukturen in O/W-Emulsionen.
